# EUROPEAN PATENT SPECIFICATION

(11) **EP 3 998 063 B1**
(45) Date of publication and mention of the grant of the patent: **02.09.2026**
(21) Application number: 19936585.9
(22) Date of filing: 08.07.2019
(51) Int. Cl.: A61K 9/107, A61K 47/26, A61K 47/44, A01N 25/04, A23D 7/00, A23L 33/10, A61K 8/81, B01J 13/00, C09D 7/63, C09D 201/00, A01N 37/00, A23D 7/005, A23L 29/10, A23L 35/00, A61K 31/045, A61K 31/12, A61K 31/122, A61K 31/337, A61K 31/405

(54) **MICROEMULSION AND A METHOD FOR PRODUCING A MICROEMULSION**
MIKROEMULSION UND VERFAHREN ZUR HERSTELLUNG DER MIKROEMULSION
MICROÉMULSION ET PROCÉDÉ DE PRODUCTION DE LA MICROÉMULSION

(43) Date of publication of application: 18.05.2022
(73) Proprietor: MORESCO Corporation, Kobe-shi, Hyogo 650-0047 (JP)
(72) Inventor: MARUYAMA, Shingo, Kobe-shi, Hyogo 650-0047 (JP)
(74) Representative: Bittner, Thomas L.
(86) International application number: PCT/JP2019/026961
(87) International publication number: WO 2021/005676

(56) References cited:
- WO-A1-2018/061011
- JP-A- 2013 543 868
- JP-A- 2014 098 039
- JP-A- 2016 512 562
- JP-A- 2016 512 828
- JP-A- 2017 071 645
- MORSI NADIA ET AL: "Nanoemulsion-based electrolyte triggered in situ gel for ocular delivery of acetazolamide", EUROPEAN JOURNAL OF PHARMACEUTICAL SCIENCES, ELSEVIER AMSTERDAM, NL, vol. 104, 19 April 2017 (2017-04-19), pages 302 - 314, XP085093814, ISSN: 0928-0987, DOI: 10.1016/J.EJPS.2017.04.013

## Description

The present invention relates to a composition for preparing a microemulsion, the microemulsion, methods for producing the composition and the microemulsion, and a use of the microemulsion.

### Background

Emulsification (formation of an emulsion) is known as a method for solubilizing a poorly water-soluble substance. For example, it is known that a poorly water-soluble drug having physiological activity is improved in oral and transdermal absorption efficiency through emulsification. It is also known that the smaller the particle diameter of emulsified particles, the higher the absorption efficiency.

For example, document JP 2017-071645 A discloses a microemulsion obtained by diluting, with an aqueous medium, a mixture obtained by dissolving a fat-soluble functional component in a system which is composed of coconut oil and polysorbate 80 and in which a weight ratio between the coconut oil and the polysorbate 80 is 1:4 to 1:9.

In the studies according to the article MORSI NADIA et al. "Nanoemulsion-based electrolyte triggered in situ gel for ocular delivery of acetazolamide", European Journal of Pharmaceutical Sciences, Elsevier, 19 April 2017, vol. 104, pages 302-314, the antiglaucoma drug, acetazolamide, was formulated as an ion induced nanoemulsion-based in situ gel for ocular delivery aiming a sustained drug release and an improved therapeutic efficacy. Different acetazolamide loaded nanoemulsion formulations were prepared using peanut oil, tween 80 and/or cremo-phor EL as surfactant in addition to transcutol P or propylene glycol as cosurfactant.

Document WO 2018 / 061 011 A1 provides methods for extraction of various agents, e.g., active agent, from a plant source, specifically with the use of extraction formulations which are based on micellar liquid systems.

### Summary

### Technical Problem

Unfortunately, the conventional technique as described above has the problem that the particle diameter of the emulsified particles of the microemulsion is still large, the particle diameter distribution is wide, and the uniformity in particle diameter is insufficient.

It is an object of the present invention to provide: a microemulsion having emulsified particles of a small particle diameter, having a narrow particle diameter distribution, and having improved uniformity in particle diameter; a composition for preparing such a microemulsion; methods for producing the microemulsion and the composition; and an object using the microemulsion.

### Solution to Problem

The inventors of the present invention have diligently studied to solve the above problem and have accomplished the present invention by finding, through their own study, that the above problem can be solved by providing a microemulsion according to claim 1, a pharmaceutical product according to claim 2, and a method for producing a microemulsion according to claim 3. A further embodiment is disclosed in dependent claim 4.

An embodiment of the present invention includes the following arrangements:
A composition for preparing a microemulsion, the composition including: a surfactant component which contains at least one selected from polysorbate 80 and a polyoxyethylene castor oil; an oil; and a poorly water-soluble functional component, wherein a weight ratio of the surfactant component to the oil is not less than 9.20 and not more than 240, and a weight ratio of the surfactant component to a sum of the oil and the poorly water-soluble functional component is not less than 5.20 and not more than 200.

When LogP of the poorly water-soluble functional component is not less than 4.2, a weight ratio of the oil to the poorly water-soluble functional component is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000.

The microemulsion includes the composition and an aqueous medium.

A pharmaceutical product, a functional food, or a cosmetic product may be provided, each including the microemulsion.

The method includes a mixing step of mixing a surfactant component which contains at least one selected from polysorbate 80 and a polyoxyethylene castor oil, an oil, and a poorly water-soluble functional component, wherein a weight ratio of the surfactant component to the oil is not less than 9.20 and not more than 240, and a weight ratio of the surfactant component to a sum of the oil and the poorly water-soluble functional component is not less than 5.20 and not more than 200.

When LogP of the poorly water-soluble functional component is not less than 4.2, a weight ratio of the oil to the poorly water-soluble functional component is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000.

The method includes: a mixing step of mixing a surfactant component which contains at least one selected from polysorbate 80 and a polyoxyethylene castor oil, an oil, and a poorly water-soluble functional component; and a step of mixing a composition obtained in the mixing step and an aqueous medium, wherein a weight ratio of the surfactant component to the oil is not less than 9.20 and not more than 240, and a weight ratio of the surfactant component to a sum of the oil and the poorly water-soluble functional component is not less than 5.20 and not more than 200.

When LogP of the poorly water-soluble functional component is not less than 4.2, a weight ratio of the oil to the poorly water-soluble functional component is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000. The microemulsion may have an average particle diameter of not more than 18 nm, having a particle size distribution index of not more than 0.14, and being a monodisperse microemulsion which does not contain particles of not less than 100 nm.

### Advantageous Effects of Invention

According to an aspect of the present invention, it is possible to provide: a microemulsion having emulsion particles of a small particle diameter, having a narrow particle diameter distribution, and having improved uniformity in particle diameter; a composition for preparing such a microemulsion; methods for producing the microemulsion and the composition; and an object using the microemulsion.

### Description of Embodiments

The following will describe embodiments of the present invention in detail. Unless otherwise specified herein, "A to B" which indicates a numerical range means "not less than A and not more than B".

### [1. Composition for preparing microemulsion]

The present invention is directed to a microemulsion. Here, in the present specification, the "microemulsion" refers to an optically transparent or semi-transparent dispersion system of minute droplets that is made of water, oil, and a surfactant and that has a particle diameter of several nanometers to several tens of nanometers. The minute droplets correspond to micelles described later and are emulsion particles (emulsified particles) of a microemulsion. The microemulsion is formed by adding an aqueous medium to the composition for preparing a microemulsion in accordance with an embodiment of the present invention.

The microemulsion in accordance with the present invention (hereinafter also referred to as "microemulsion preparation composition" in the present specification) contains: a surfactant component which contains at least one selected from polysorbate 80 and a polyoxyethylene castor oil; an oil; and a poorly water-soluble functional component, wherein a weight ratio of the surfactant component to the oil is not less than 9.20 and not more than 240, and a weight ratio of the surfactant component to a sum of the oil and the poorly water-soluble functional component is not less than 5.20 and not more than 200. A microemulsion in accordance with the present invention contains: a surfactant component which contains at least one selected from polysorbate 80 and a polyoxyethylene castor oil; an oil; and a poorly water-soluble functional component, wherein: a weight ratio of the surfactant component to the oil is not less than 9.20 and not more than 240; a weight ratio of the surfactant component to a sum of the oil and the poorly water-soluble functional component is not less than 5.20 and not more than 200; when LogP of the poorly water-soluble functional component is not less than 4.2, a weight ratio of the oil to the poorly water-soluble functional component is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000.

### (Surfactant component)

A microemulsion in accordance with the present invention contains a surfactant component which contains at least one selected from polysorbate 80 and a polyoxyethylene castor oil.

According to the invention, the microemulsion preparation composition contains at least one selected from polysorbate 80 and a polyoxyethylene castor oil, as a main component of the surfactant component contained in the composition. Note here that the "main component" is preferably not less than 80% by weight, more preferably not less than 85% by weight, even more preferably not less than 90% by weight, particularly preferably not less than 95% by weight, and most preferably 100% by weight, relative to the total weight of the surfactant component contained in the microemulsion preparation composition.

In an embodiment of the present invention, the polyoxyethylene castor oil is a compound obtained by addition polymerization of ethylene oxide to a castor oil. An average addition mole number of the ethylene oxide, although not particularly limited, is preferably 2 to 100, and more preferably 10 to 50. Examples of the polyoxyethylene castor oil include NIKKOL CO-3, NIKKOL CO-10 (Nikko Chemicals), EMALEX C-20, EMALEX C-30, EMALEX C-40, EMALEX C-50 (Nihon Emulsion), and Kolliphor EL (BASF). One of these polyoxyethylene castor oils can be used alone. Alternatively, two or more of these polyoxyethylene castor oils can be used in combination. The polyoxyethylene castor oil is more preferably Kolliphor EL.

In an embodiment of the present invention, the surfactant component only needs to contain at least one selected from the polysorbate 80 and the polyoxyethylene castor oil. In a case where the surfactant component contains both the polysorbate 80 and the polyoxyethylene castor oil, a proportion in which the polysorbate 80 and the polyoxyethylene castor oil are used is not particularly limited, and the ratio between the polysorbate 80 and the polyoxyethylene castor oil is 1:99 to 99:1 on a weight basis.

Since the microemulsion preparation composition contains the surfactant component which contains at least one selected from the polysorbate 80 and the polyoxyethylene castor oil in the above range, it is possible to prepare, from such a microemulsion preparation composition, a microemulsion that has a small emulsion particle diameter, has a narrow particle diameter distribution, and has improved uniformity in particle diameter.

### (Oil)

The microemulsion preparation composition in accordance with an embodiment of the present invention contains an oil. The oil is not particularly limited, provided that it can be commonly used for edible, pharmaceutical, and cosmetic purposes. Examples of the oil include: naturally derived oils such as avocado oil, olive oil, sesame oil, almond oil, bergamot oil, camellia oil, evening primrose oil, macadamia nut oil, corn oil, rape-seed oil, persic oil, wheat germ oil, camellia sasanqua oil, castor oil, linseed oil, safflower oil, cottonseed oil, perilla oil, soybean oil, peanut oil, tea seed oil, kaya oil, rice bran oil, tung oil, canola oil, red flower oil, kukui nut oil, grape seed oil, hazelnut oil, sunflower oil, rose hip oil, pistachio nut oil, coconut oil, and vegetable squalane; and synthetic oils such as medium chain fatty acid triglyceride, isopropyl myristate, octyldodecyl myristate, and liquid paraffin. Above all, the naturally derived oils are more preferably vegetable oils. Further, from the viewpoint of absorption into a living body and solubility of functional components such as pharmaceutical drugs, the oil is more preferably soybean oil, medium chain fatty acid triglyceride, coconut oil, bergamot oil, rose hip oil, and almond oil, and more preferably soybean oil and medium chain fatty acid triglyceride. One of the above oils can be used alone. Alternatively, two or more of the above oils can be used in combination. The oil is an oil having a freezing point of not higher than 25°C. Vegetable oils containing a large amount of wax components, such as palm oil and jojoba oil, beef tallow, lard, and the like all of which can solidify at room temperature (25°C), are not preferable since they may impair the stability of an emulsion.

The above-described oil is preferably used since it makes it possible to obtain a stable microemulsion in which emulsion particles have a small particle diameter.

### (Poorly water-soluble functional component)

A microemulsion preparation composition in accordance with the present invention contains a poorly water-soluble functional component.

In the present specification, "poorly water-soluble" means "difficult to dissolve", "extremely difficult to dissolve", and "practically insoluble" as defined in the Japanese Pharmacopoeia, and refers to having solubility in water at 20°C of not more than 10 mg/ml. More preferably, "poorly water-soluble" means "extremely difficult to dissolve" and "practically insoluble" as defined in the Japanese Pharmacopoeia, and refers to having solubility in water at 20°C of not more than 1 mg/ml. The poorly water-soluble functional component has a low degree of solubility in water. Thus, when dissolved in the oil to form a microemulsion, the poorly water-soluble functional component can be dispersed in an aqueous medium. The poorly water-soluble functional component is preferably poorly water-soluble and fat-soluble.

Further, in the present specification, the "functional component" is not particularly limited, provided that it is a component having some kind of function, and includes, for example, a component that is absorbed in a living body and brings about various functional effects, a component that is contained in a coating agent and brings about various functional effects, and the like component.

The above-mentioned component that is absorbed in a living body and brings about various functional effects includes a component that has pharmacological activity for use in inhibiting, diagnosing, alleviating, treating, curing, or preventing a disease or disease condition, a component that provides a predetermined nutritional or health benefit to a living body, and the like component. In particular, the poorly water-soluble functional component which is inferior in bioabsorbability as it is has a remarkable effect of providing the composition of the present invention.

The functional component having pharmacological activity is a component used in pharmaceutical products and the like. Examples of the poorly water-soluble functional component having pharmacological activity include, but are not particularly limited to, paclitaxel, cyclosporin, indomethacin, terfenadine, furosemide, acetazolamide, colistin, mebendazole, albendazole, nilotinib, lormetazepam, bromazepam, and the like.

Examples of the component that provides a nutritional or health benefit include components used in foods for special dietary uses, foods with health claims (foods for specified health uses, foods with nutrient function claims), functional foods, nutrition-supplement foods, health-supplement foods, nutrient-enriched foods, nutrient-adjusted foods, supplements, and the like. Examples of such a poorly water-soluble functional component include coenzyme Q10 (herein also referred to as "CoQ10"), curcumin, tocotrienol, testosterone, menthol, and carotenoids (α-carotene, β-carotene, lutein, lycopene, astaxanthin, zeaxanthin, cryptoxanthin, fucoxanthin, xanthophyll, and the like), resveratrol, docosahexaenoic acid (DHA), eikosapentaenoic acid (EPA), fat-soluble vitamins (vitamin A, vitamin D, vitamin E (tocopherol), vitamin K), sesamin, α-lipoic acid, saw palmetto extract (oleic acid, lauric acid, myristic acid, linoleic acid, palmitic acid), St. John's wort (hypericin), royal jelly (decenoic acid), hesperidin, nobiletin, quercetin, kaempferol, myricitrin, catechin, daidzein, glycitein, genistein, myricetin, stilbene, and the like, and combinations of two or more of these compounds.

Alternatively, examples of the component that provides a nutritional or health benefit include components used in cosmetic products such as skin improving components, moisturizing components, anti-aging components, hair growth components, and trichogenous components. Examples of such a poorly water-soluble functional component include coenzyme Q10, curcumin, tocotrienol, carotenoids (α-carotene, β-carotene, lutein, lycopene, astaxanthin, zeaxanthin, cryptoxanthin, fucoxanthin, xanthophyll, and the like), resveratrol, docosahexaenoic acid (DHA), eikosapentaenoic acid (EPA), fat-soluble vitamins (vitamin A, vitamin D, vitamin E (tocopherol), vitamin K), sesamin, α-lipoic acid, and combinations of two or more of these compounds.

Further, examples of the functional component also include components, such as a color-developing component, used as a coating agent in an industrial field such as printing. Examples of such a poorly water-soluble functional component include coenzyme Q10, curcumin, carotenoids (α-carotene, β-carotene, lutein, lycopene, astaxanthin, zeaxanthin, cryptoxanthin, fucoxanthin, xanthophyll, and the like), and the like, and combinations of two or more of these compounds.

Further, the functional component also includes components used as pesticides such as dichlorodiphenyltrichloroethane (DDT), tebufenpyrad, fenoxycarb, and the like.

### (Weight ratio between individual components)

A microemulsion preparation composition in accordance with the present invention satisfies the conditions (i) and (ii) below to bring about the effect of making it possible to realize, when an aqueous medium is mixed thereto, a microemulsion having emulsion particles of a small particle diameter, having a narrow particle diameter distribution, and having improved uniformity in particle diameter of the emulsion particles.
(i) The weight ratio of the surfactant component containing at least one selected from the polysorbate 80 and the polyoxyethylene castor oil to the oil (the surfactant component containing at least one selected from the polysorbate 80 and the polyoxyethylene castor oil / the oil) is not less than 9.20 and not more than 240.
(ii) The weight ratio of the surfactant component containing at least one selected from the polysorbate 80 and the polyoxyethylene castor oil to the sum of the oil and the poorly water-soluble functional component (the surfactant component containing at least one selected from the polysorbate 80 and the polyoxyethylene castor oil / (the oil + the poorly water-soluble functional component)) is not less than 5.20 and not more than 200.

Further, a microemulsion preparation composition in accordance with the present invention satisfies not only the above conditions (i) and (ii) but also the condition (iii) below to bring about the effect of making it possible to realize, when an aqueous medium is mixed thereto, a microemulsion having emulsion particles of a small particle diameter, having a narrow particle diameter distribution, having improved uniformity in particle diameter of the emulsion particles, and having excellent stability.

(iii) When LogP of the poorly water-soluble functional component is not less than 4.2, the weight ratio of the oil to the poorly water-soluble functional component (the oil / the poorly water-soluble functional component) is not less than [-0.38 × (the LogP of the poorly water-soluble functional component) + 5.169] and not more than 2000, and when the LogP of the poorly water-soluble functional component is less than 4.2, the weight ratio of the oil to the poorly water-soluble functional component (the oil / the poorly water-soluble functional component) is not less than [-4.955 × (the LogP of the poorly water-soluble functional component) + 23.56] and not more than 2000.

The weight ratio of the surfactant component to the oil only needs to be not less than 9.20 and not more than 240, but a lower limit of the weight ratio of the surfactant component to the oil is more preferably 9.40, even more preferably 9.60, further more preferably 10, and particularly preferably 20.

Further, the weight ratio of the surfactant component to the sum of the oil and the poorly water-soluble functional component only needs to be not less than 5.20 and not more than 200, but a lower limit of the weight ratio of the surfactant component to the sum of the oil and the poorly water-soluble functional component is more preferably 5.8, even more preferably 6.0, and further more preferably 8.0.

With a microemulsion preparation composition in which (the surfactant component containing at least one selected from the polysorbate 80 and the polyoxyethylene castor oil / the oil) is not less than 9.20, and (the surfactant component containing at least one selected from the polysorbate 80 and the polyoxyethylene castor oil / (the oil + the poorly water-soluble functional component)) is not less than 5.20, it is possible to obtain a microemulsion having emulsion particles of a small particle diameter, having a narrow particle diameter distribution, and having improved uniformity in particle diameter of the emulsion particles. Further, it is preferable that (the surfactant component containing at least one selected from the polysorbate 80 and the polyoxyethylene castor oil / the oil) is not less than 9.60, and [the surfactant component containing at least one selected from the polysorbate 80 and the polyoxyethylene castor oil / (the oil + the poorly water-soluble functional component)] is not less than 5.20, since such a composition not only brings about the above effect, but also enables the emulsion particles to have a smaller particle diameter.

When the LogP of the poorly water-soluble functional component is not less than 4.2, the weight ratio of the oil to the poorly water-soluble functional component (the oil / the poorly water-soluble functional component) only needs to be not less than [-0.38 × (the LogP of the poorly water-soluble functional component) + 5.169] and not more than 2000, but a lower limit of the weight ratio of the oil to the poorly water-soluble functional component is more preferably not less than [-0.38 × (the LogP of the poorly water-soluble functional component) + 5.9], and even more preferably not less than [-0.38 × (the LogP of the poorly water-soluble functional component) + 6.7]. It is preferable that when the LogP of the poorly water-soluble functional component is not less than 4.2, (the oil / the poorly water-soluble functional component) is not less than [-0.38 × (the LogP of the poorly water-soluble functional component) + 5.169], since such a condition provides a sufficient amount of oil for stably dissolving the poorly water-soluble functional component and thus makes it possible to realize a microemulsion having excellent stability.

When the LogP of the poorly water-soluble functional component is less than 4.2, the weight ratio of the oil to the poorly water-soluble functional component (the oil / the poorly water-soluble functional component) only needs to be not less than [-4.955 × (the LogP of the poorly water-soluble functional component) + 23.56] and not more than 2000, but a lower limit of the weight ratio of the oil to the poorly water-soluble functional component is more preferably not less than [-4.955 × (the LogP of the poorly water-soluble functional component) + 26], and even more preferably not less than [-4.955 × (the LogP of the poorly water-soluble functional component) + 29]. It is preferable that when the LogP of the poorly water-soluble functional component is not less than 4.2, (the oil / the poorly water-soluble functional component) is not less than [-4.955 × (the LogP of the poorly water-soluble functional component) + 23.56], since such a condition provides a sufficient amount of oil for stably dissolving the poorly water-soluble functional component and thus makes it possible to realize a microemulsion having excellent stability.

Here, LogP is a common logarithmic value of a 1-octanol-water or 1-octanol-buffer partition coefficient of a chemical substance. In the present specification, LogP is a value measured by a flask shaking method based on OECD GUIDELINE FOR THE TESTING OF CHEMICALS, "Partition Coefficient (n-octanol/water): Shake Flask Method". A chemical substance having a higher LogP has a higher degree of lipophilicity. In other words, a chemical substance having a higher LogP has a higher degree of fat-solubility.

In the microemulsion in accordance with the present invention, the oil acts as a solvent for the poorly water-soluble functional component. The inventors of the present invention focused on the lipophilicity of the poorly water-soluble functional component. That is, the inventors of the present invention thought that the polarity of the poorly water-soluble functional component varies depending on the type of the poorly water-soluble functional component, and the amount of oil required to stably dissolve the poorly water-soluble functional component also varies accordingly. Then, the inventors have found, from various study results, that a stable emulsion could be obtained by setting the weight ratio of the oil to the poorly water-soluble functional component (the oil / the poorly water-soluble functional component) to fall within a range that satisfies the above condition (iii).

### [2. Microemulsion]

A microemulsion in accordance with an embodiment of the present invention contains the microemulsion preparation composition and an aqueous medium.

In the present specification, the "aqueous medium" is not particularly limited, provided that it is a medium which contains water. Examples of the aqueous medium include water, bubble water, fruit juice, vegetable juice, soft drinks, milk, yogurt drinks, soy milk, tea drinks, sports drinks, nutrition-supplement drinks, and the like. The "water" can be, for example, so-called water such as ultrapure water (MilliQ (registered trademark) water), distilled water, and ion-exchanged water, and is also intended to include various buffer solutions such as a phosphate buffer solution and physiological saline. One of the above aqueous media can be used alone. Alternatively, two or more of the above aqueous media can be used in combination.

The amount of the aqueous medium contained in a microemulsion in accordance with an embodiment of the present invention is not particularly limited, provided that it is an amount that allows the microemulsion to be formed. The aqueous medium is contained in an amount of preferably 50% by weight to 99.9% by weight, more preferably 60% by weight to 95% by weight, and even more preferably 70% by weight to 90% by weight, relative to the total weight of the microemulsion.

The amount of the aqueous medium contained in the microemulsion is not less than 50% by weight, since such an amount makes it possible to suitably form a microemulsion. Further, the amount of the aqueous medium contained in the microemulsion is preferably not more than 99.9% by weight, since such an amount does not cause an excessively low concentration of the poorly water-soluble functional component.

In a microemulsion in accordance with the present invention, the surfactant component containing at least one selected from the polysorbate 80 and the polyoxyethylene castor oil and the oil form micelles. Specifically, the poorly water-soluble functional component is dissolved in the oil, and the oil is surrounded by the surfactant component with its hydrophobic group positioned inside, so that micelles are formed. The micelles are emulsion particles of the microemulsion.

The emulsion particles have an average particle diameter of preferably not more than 18 nm. The emulsion particles preferably have an average particle diameter of not more than 18 nm since such an average particle diameter makes it possible to improve the efficiency in absorption of the poorly water-soluble functional component into a living body. Further, a small average particle diameter of the emulsion particles makes it possible to obtain a transparent microemulsion. Note here that the average particle diameter of the emulsion particles refers to a value that is measured with use of Zetasizer Nano ZS (available from Malvern Institutes) in the measurement mode "size-small-vol-cell×1.SOP".

Further, the PDI (particle size distribution index) of the emulsion particles is preferably not more than 0.14. The PDI (particle size distribution index) of the emulsion particles is preferably not more than 0.14 since such a PDI makes it possible to realize a microemulsion that contains emulsion particles which have excellent absorbability into a living body and many of which have an average particle diameter of not more than 18 nm and that has excellent stability. Note here that the PDI (particle size distribution index) of the emulsion particles refers to a value that is measured with use of Zetasizer Nano ZS (available from Malvern Institutes) in the "size-small-vol-cell×1.SOP".

Further, the microemulsion is preferably a monodisperse dispersion that does not contain particles of not less than 100 nm (subpeaks). The microemulsion is preferably a monodisperse dispersion that does not contain particles of not less than 100 nm (subpeaks) and that has improved uniformity in particle diameter of the emulsion particles, since it is possible to realize a microemulsion that is transparent, has excellent absorbability into a living body, and has excellent stability.

Further, the microemulsion is an oil-in-water type microemulsion that has excellent stability and that exists in a stable state at 25°C for at least 14 days.

### [3. Method for producing microemulsion preparation composition]

A method for producing a microemulsion preparation composition in accordance with an embodiment of the present invention only needs to include a mixing step of mixing the above-described surfactant component containing at least one selected from polysorbate 80 and a polyoxyethylene castor oil, the above-described oil, and the above-described poorly water-soluble functional component. Here, the mixing of the surfactant component, the oil, and the poorly water-soluble functional component is performed such that a weight ratio of the surfactant component to the oil is not less than 9.20 and not more than 240, and a weight ratio of the surfactant component to the sum of the oil and the poorly water-soluble functional component is not less than 5.20 and not more than 200.

A method for producing a microemulsion preparation composition in accordance with an embodiment of the present invention only needs to include a mixing step of mixing the surfactant component, the oil, and the poorly water-soluble functional component, and here, more preferably, the mixing of the surfactant component, the oil, and the poorly water-soluble functional component is performed such that: a weight ratio of the surfactant component to the oil is not less than 9.20 and not more than 240; a weight ratio of the surfactant component to the sum of the oil and the poorly water-soluble functional component is not less than 5.20 and not more than 200; when LogP of the poorly water-soluble functional component is not less than 4.2, a weight ratio of the oil to the poorly water-soluble functional component (the oil / the poorly water-soluble functional component) is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component (the oil / the poorly water-soluble functional component) is not less than [-4.955 × (the LogP of the poorly water-soluble functional component) + 23.56] and not more than 2000.

In the mixing step, the order in which the surfactant component, the oil, and the poorly water-soluble functional component are mixed is not particularly limited. The surfactant component, the oil, and the poorly water-soluble functional component may be all mixed at the same time. Alternatively, the poorly water-soluble functional component may be mixed after the surfactant component and the oil are mixed, the oil may be mixed after the surfactant component and the poorly water-soluble functional component are mixed, or the surfactant component may be mixed after the oil and the poorly water-soluble functional component are mixed.

A method of mixing the individual components described above is not particularly limited, provided that the method can uniformly dissolve the surfactant component, the oil, and the poorly water-soluble functional component. Examples of the method include a method of mixing and stirring the individual components. In the present invention, the individual components can be uniformly dissolved without applying a particularly strong shearing force. For example, the individual components can be uniformly dissolved by stirring them with a magnetic stirrer.

Further, a temperature at which the individual components are mixed is not particularly limited, but a liquid temperature is more preferably not lower than 20°C and not higher than 90°C, and even more preferably not lower than 30°C and not higher than 75°C.

### [4. Method for producing microemulsion]

A method for producing a microemulsion in accordance with an embodiment of the present invention only needs to include: a mixing step of mixing the above-described surfactant component which contains at least one selected from polysorbate 80 and a polyoxyethylene castor oil, the above-described oil, and the above-described poorly water-soluble functional component; and an aqueous medium adding step of mixing a composition obtained in the mixing step and an aqueous medium.

The mixing step has been described under "3. Method for producing microemulsion preparation composition" above.

The step of mixing the composition obtained in the mixing step and the aqueous medium is a step of adding the aqueous medium to the composition obtained in the mixing step. This step may involve adding the aqueous medium in its entirety at a time or in fractional amounts while the mixture is being stirred.

A method of mixing the composition obtained in the mixing step and the aqueous medium is not particularly limited, provided that the method can disperse the composition as emulsion particles in the aqueous medium. Examples of the method include a method of mixing and stirring the composition and the aqueous medium. In this step as well, the composition can be dispersed as emulsion particles in the aqueous medium without applying a particularly strong shearing force. For example, the composition and the aqueous medium can be stirred with a magnetic stirrer.

Further, the temperature at which the composition and the aqueous medium are mixed is not particularly limited, but a liquid temperature is more preferably not lower than 20°C and not higher than 90°C, and even more preferably not lower than 30°C and not higher than 75°C.

### [4. Use of microemulsion]

The present invention also includes a pharmaceutical product, a functional food, a cosmetic product, a coating agent, or a pesticide, each containing a microemulsion in accordance with an embodiment of the present invention.

The present invention also encompasses, in its technical scope, any embodiment derived by combining technical means disclosed in differing embodiments.

### Examples

The following will describe the present invention in greater detail on the basis of Examples.

### [Evaluation method]

Microemulsions obtained in Examples and Comparative Examples were evaluated by the following methods.

(Measurements of average particle diameter, polydispersity index (PDI), and particle diameter distribution (presence or absence of subpeak) of emulsion particles in microemulsion)

The microemulsions obtained in Examples and Comparative Examples were each diluted 20-fold with ion-exchanged water to prepare samples for measurement. With use of Zetasizer Nano ZS (available from Malvern Panalytical Ltd.), the PDI was measured in the measurement mode "size-small-vol-cell×1.SOP". Further, the particle diameter of the emulsion particles was measured on the basis of a Z-average value. Three measurements were made with use of the same samples. The average was used as the average particle diameter of the emulsion particles.

The presence or absence of subpeaks was determined from a particle diameter distribution graph obtained as the results of the measurement. If there are any subpeaks, the measurement results are automatically outputted as Peak-2, Peak-3, etc.

### (Transparency)

The microemulsions obtained in Examples and Comparative Examples were visually observed, and a case where the microemulsions were uniform and clear was regarded as "transparent", and a case where a liquid layer was separated into two layers and a case where a deposit was suspended were regarded as "opaque".

### (Stability/precipitation)

The microemulsions obtained in Examples and Comparative Examples were sealed in a colorless and transparent glass bottle and allowed to stand at a temperature of 25°C, and a period of time from the start of standing to the occurrence of precipitation was measured.

### [Preparation of microemulsion preparation composition and microemulsion]

### (Example 1)

In a 10 ml beaker, CoQ10 (available from FUJIFILM Wako Pure Chemical Corporation; Ubiquinone-10) as a poorly water-soluble functional component, a soybean oil (available from FUJIFILM Wako Pure Chemical Corporation; soybean oil) as an oil, and polysorbate 80 (available from Kao Corporation; EMASOL O-120V) were weighed so as to have a weight ratio shown in Table 1. The weighed mixture in the beaker was stirred at 60°C with a magnetic stirrer until the whole of the poorly water-soluble functional component was uniformly dissolved by a magnetic stirring bar, so that a microemulsion preparation composition, which was a viscous liquid, was obtained. To this microemulsion preparation composition, ion-exchanged water was added so as to have the weight ratio shown in Table 1, and the mixture was stirred at 60°C for another 1 to 3 minutes. After it was visually confirmed that there was no uneven undissolved residue or lump, the mixture was cooled to room temperature in an ice water tank to obtain a microemulsion. The transparency and stability of the obtained microemulsion, and the average particle diameter, PDI, and presence or absence of subpeaks of emulsion particles in the microemulsion were evaluated. The results are shown in Table 1.

### (Examples 2 to 12)

Microemulsions were each prepared in the same manner as in Example 1 except that the weight ratio of CoQ10, the soybean oil, the polysorbate 80, and the ion-exchanged water was changed to the weight ratios shown in Table 1. The obtained microemulsions were evaluated. The results are shown in Table 1.

**[Table 1]**

| | Example 1 | Example 2 | Example 3 | Example 4 | Example 5 | Example 6 |
|---|---|---|---|---|---|---|
| Water (% by weight) | 72.5 | 71.6 | 71.4 | 72.6 | 75.88 | 74.8 |
| Polysorbate 80 (% by weight) | 24.0 | 24.0 | 24.0 | 23.0 | 24.00 | 24.0 |
| Soybean oil (% by weight) | 2.5 | 2.5 | 2.5 | 2.5 | 0.10 | 1.0 |
| CoQ10 (% by weight) | 1.0 | 1.9 | 2.1 | 1.9 | 0.02 | 0.2 |
| Polysorbate 80/soybean oil | 9.60 | 9.60 | 9.60 | 9.20 | 240 | 24.0 |
| Polysorbate 80/(soybean oil + CoQ10) | 6.90 | 5.33 | 5.22 | 5.23 | 200 | 20.0 |
| LogP of CoQ10 | 11 | 11 | 11 | 11 | 11 | 11 |
| -0.38 × [LogP] + 5.169 | 0.99 | 0.99 | 0.99 | 0.99 | 0.99 | 0.99 |
| Soybean oil/CoQ10 | 2.50 | 1.32 | 1.19 | 1.32 | 5.00 | 5.00 |
| Transparency | Transparent | Transparent | Transparent | Transparent | Transparent | Transparent |
| Stability/precipitation (days) | > 100 | > 95 | > 14 | > 14 | > 14 | > 14 |
| Average particle diameter (nm) | 10.74 | 12.54 | 11.84 | 13.17 | 8.058 | 8.563 |
| Particle size distribution index PDI | 0.113 | 0.063 | 0.134 | 0.087 | 0.128 | 0.134 |
| Subpeak of average particle diameter of not less than 100 nm (%) | 0 | 0 | 0 | 0 | 0 | 0 |

**[Table 1] (Cont.)**

| | Example 7 | Example 8 | Example 9 | Example 10 | Example 11 | Example 12 |
|---|---|---|---|---|---|---|
| Water (% by weight) | 70.25 | 71.5 | 71.7 | 71.9 | 72.3 | 72.08 |
| Polysorbate 80 (% by weight) | 25.00 | 24.0 | 24.0 | 24.0 | 24.0 | 24.00 |
| Soybean oil (% by weight) | 2.65 | 2.5 | 2.5 | 2.5 | 2.5 | 1.80 |
| CoQ10 (% by weight) | 2.10 | 2.0 | 1.8 | 1.6 | 1.2 | 2.11 |
| Polysorbate 80/soybean oil | 9.43 | 9.60 | 9.60 | 9.60 | 9.60 | 13.33 |
| Polysorbate 80/(soybean oil + CoQ10) | 5.26 | 5.33 | 5.58 | 5.85 | 6.49 | 6.14 |
| LogP of CoQ10 | 11 | 11 | 11 | 11 | 11 | 11 |
| -0.38 × [LogP] + 5.169 | 0.99 | 0.99 | 0.99 | 0.99 | 0.99 | 0.99 |
| Soybean oil/CoQ10 | 1.26 | 1.25 | 1.39 | 1.56 | 2.08 | 0.85 |
| Transparency | Transparent | Transparent | Transparent | Transparent | Transparent | Transparent |
| Stability/precipitation (days) | > 14 | > 14 | > 14 | > 14 | > 14 | Deposition on 10th day |
| Average particle diameter (nm) | 12.73 | 12.83 | 12.12 | 11.79 | 10.54 | 11.02 |
| Particle size distribution index PDI | 0.097 | 0.096 | 0.093 | 0.110 | 0.086 | 0.069 |
| Subpeak of average particle diameter of not less than 100 nm (%) | 0 | 0 | 0 | 0 | 0 | 0 |

### (Comparative Examples 1 to 10)

Microemulsions were each prepared in the same manner as in Example 1 except that the weight ratio of CoQ10, the soybean oil, the polysorbate 80, and the ion-exchanged water was changed to the weight ratios shown in Table 2. The obtained microemulsions were evaluated. The results are shown in Table 2.

**[Table 2]**

| | Comparative Example 1 | Comparative Example 2 | Comparative Example 3 | Comparative Example 4 | Comparative Example 5 |
|---|---|---|---|---|---|
| Water (% by weight) | 71.3 | 71.8 | 72.4 | 73.1 | 73.0 |
| Polysorbate 80 (% by weight) | 24.0 | 23.5 | 23.0 | 22.5 | 22.5 |
| Soybean oil (% by weight) | 2.5 | 2.5 | 2.5 | 2.5 | 2.5 |
| CoQ10 (% by weight) | 2.2 | 2.2 | 2.1 | 1.9 | 2.0 |
| Polysorbate 80/soybean oil | 9.60 | 9.40 | 9.20 | 9.00 | 9.00 |
| Polysorbate 80/(soybean oil + CoQ10) | 5.11 | 5.00 | 5.00 | 5.11 | 5.00 |
| LogP of CoQ10 | 11 | 11 | 11 | 11 | 11 |
| -0.38 × [LogP] + 5.169 | 0.99 | 0.99 | 0.99 | 0.99 | 0.99 |
| Soybean oil/CoQ10 | 1.14 | 1.14 | 1.19 | 1.32 | 1.25 |
| Transparency | Transparent | Transparent | Transparent | Transparent | Transparent |
| Stability/precipitation (days) | > 14 | > 14 | > 14 | > 14 | > 14 |
| Average particle diameter (nm) | 14.14 | 17.08 | 12.75 | 13.92 | 17.68 |
| Particle size distribution index PDI | 0.201 | 0.223 | 0.185 | 0.223 | 0.211 |
| Subpeak of average particle diameter of not less than 100 nm (%) | 2.6 | 3.4 | 2.2 | 2.7 | 2.6 |

**[Table 2] (Cont.)**

| | Comparative Example 6 | Comparative Example 7 | Comparative Example 8 | Comparative Example 9 | Comparative Example 10 |
|---|---|---|---|---|---|
| Water (% by weight) | 71.5 | 73.7 | 71.0 | 71.5 | 71.5 |
| Polysorbate 80 (% by weight) | 24.0 | 22.0 | 24.0 | 24.0 | 24.0 |
| Soybean oil (% by weight) | 3.5 | 2.5 | 3.0 | 3.5 | 4.0 |
| CoQ10 (% by weight) | 1.0 | 1.8 | 2.0 | 1.0 | 0.5 |
| Polysorbate 80/soybean oil | 9.00 | 8.80 | 8.00 | 6.86 | 6.00 |
| Polysorbate 80/(soybean oil + CoQ10) | 5.33 | 5.12 | 4.80 | 5.33 | 5.33 |
| LogP of CoQ10 | 11 | 11 | 11 | 11 | 11 |
| -0.38 × [LogP] + 5.169 | 0.99 | 0.99 | 0.99 | 0.99 | 0.99 |
| Soybean oil/CoQ10 | 3.50 | 1.39 | 1.50 | 3.50 | 8.00 |
| Transparency | Transparent | Transparent | Transparent | Transparent | Transparent |
| Stability/precipitation (days) | > 14 | > 14 | > 95 | > 14 | > 14 |
| Average particle diameter (nm) | 14.24 | 13.99 | 13.47 | 14.24 | 15.32 |
| Particle size distribution index PDI | 0.181 | 0.198 | 0.172 | 0.181 | 0.230 |
| Subpeak of average particle diameter of not less than 100 nm (%) | 1.3 | 2.4 | 3.3 | 1.3 | 1.6 |

### (Examples 13 to 16 and Comparative Examples 11 and 12)

Microemulsions were each prepared in the same manner as in Example 1 except that oils shown in Table 3 were used as the oil, and the weight ratio of CoQ10, the oil, the polysorbate 80, and the ion-exchanged water was changed to the weight ratios shown in Table 3. The obtained microemulsions were evaluated by the following method. The results are shown in Table 3.

In Examples 13 to 16 and Comparative Examples 11 and 12, the following oils were used as the oil.

Coconut oil (available from FUJIFILM Wako Pure Chemical Corporation; Coconut oil) Bergamot oil (available from FUJIFILM Wako Pure Chemical Corporation; bergamot oil) Rose hip oil (available from FUJIFILM Wako Pure Chemical Corporation; rose hip oil) Almond oil (available from FUJIFILM Wako Pure Chemical Corporation; almond oil)

**[Table 3]**

| | | Example 13 | Example 15 | Example 16 | Comparative Example 11 | Comparative Example 12 |
|---|---|---|---|---|---|---|
| Water (% by weight) | | 71.5 | 71.5 | 71.5 | 81.5 | 86.5 |
| Polysorbate 80 (% by weight) | | 24.0 | 24.0 | 24.0 | 15.0 | 10.0 |
| Oil | Coconut oil (% by weight) | 2.5 | | | 2.5 | 2.5 |
| | Bergamot oil (% by weight) | | | | | |
| | Rose hip oil (% by weight) | | 2.5 | | | |
| | Almond oil (% by weight) | | | 2.5 | | |
| CoQ10 (% by weight) | | 2.0 | 2.0 | 2.0 | 1.0 | 1.0 |
| Polysorbate 80/oil | | 9.60 | 9.60 | 9.60 | 6.00 | 4.00 |
| Polysorbate 80/(oil + CoQ10) | | 5.33 | 5.33 | 5.33 | 4.29 | 2.86 |
| LogP of CoQ10 | | 11 | 11 | 11 | 11 | 11 |
| -0.38 × [LogP] + 5.169 | | 0.99 | 0.99 | 0.99 | 0.99 | 0.99 |
| Oil/CoQ10 | | 1.25 | 1.25 | 1.25 | 2.50 | 2.50 |
| Transparency | | Transparent | Transparent | Transparent | Transparent | Semi-transparent |
| Stability/precipitation (days) | | > 14 | > 14 | > 14 | > 14 | > 14 |
| Average particle diameter (nm) | | 10.91 | 13.83 | 12.83 | 15.02 | 80.32 |
| Particle size distribution index PDI | | 0.089 | 0.118 | 0.096 | 0.143 | 0.556 |
| Subpeak of average particle diameter of not less than 100 nm (%) | | 0 | 0 | 0 | 0 | 14.4 |

### (Examples 17 to 38)

Microemulsions were prepared in the same manner as in Example 1 except that poorly water-soluble functional components shown in Tables 4 to 12 were used as the poorly water-soluble functional component, and the weight ratio of the poorly water-soluble functional component, the soybean oil, the polysorbate 80, and the ion-exchanged water was changed to the weight ratios shown in Tables 4 to 12. The obtained microemulsions were evaluated. The results are shown in Tables 4 to 12.

In Examples 17 to 38, the following poorly water-soluble functional components were used as the poorly water-soluble functional component.
Curcumin (available from Tokyo Chemical Industry Co., Ltd.; Curcumin) (% by weight)
Cyclosporin A (available from Tokyo Chemical Industry Co., Ltd.; Cyclosporin A)
DDT (available from Tokyo Chemical Industry Co., Ltd., DDT)
Testosterone (available from Tokyo Chemical Industry Co., Ltd.; Testosterone)
Paclitaxel (available from Tokyo Chemical Industry Co., Ltd.; Paclitaxel)
Menthol (available from Tokyo Chemical Industry Co., Ltd.; (-)-Menthol)
Tocopherol (available from Tokyo Chemical Industry Co., Ltd.; DL-α-Tocopherol)
Tocotrienol (available from Oryza Oil & Fat Chemical Co., Ltd.; ORYZA TOCOTRIENOL-90)
Indomethacin (available from Tokyo Chemical Industry Co., Ltd.; Indomethacin)

**[Table 4]**

| | Example 17 |
|---|---|
| Water (% by weight) | 73.2 |
| Polysorbate 80 (% by weight) | 24.0 |
| Soybean oil (% by weight) | 2.4 |
| Curcumin (% by weight) | 0.4 |
| Polysorbate 80/soybean oil | 10.00 |
| Polysorbate 80/(soybean oil + curcumin) | 8.57 |
| LogP of curcumin | 3.62 |
| -4.955 × [LogP] + 23.56 | 5.62 |
| Soybean oil/curcumin | 6.00 |
| Transparency | Transparent |
| Stability/precipitation (days) | > 14 |
| Average particle diameter (nm) | 12.02 |
| Particle size distribution index PDI | 0.061 |
| Subpeak of average particle diameter of not less than 100 nm (%) | 0 |

**[Table 5]**

| | Example 18 | Example 19 | Example 20 | Example 21 | Example 22 | Example 23 |
|---|---|---|---|---|---|---|
| Water (% by weight) | 73.40 | 74.00 | 72.88 | 72.68 | 72.60 | 72.24 |
| Polysorbate 80 (% by weight) | 24.00 | 24.00 | 24.02 | 24.12 | 24.06 | 24.14 |
| Soybean oil (% by weight) | 2.50 | 1.80 | 2.60 | 2.60 | 2.60 | 2.61 |
| Cyclosporin A (% by weight) | 0.10 | 0.20 | 0.50 | 0.60 | 0.74 | 1.01 |
| Polysorbate 80/soybean oil | 9.60 | 13.33 | 9.24 | 9.27 | 9.24 | 9.24 |
| Polysorbate 80/(soybean oil + cyclosporin A) | 9.23 | 12.00 | 7.75 | 7.53 | 7.19 | 6.67 |
| LogP of cyclosporin A | 4.12 | 4.12 | 4.12 | 4.12 | 4.12 | 4.12 |
| -4.955 × [LogP] + 23.56 | 3.15 | 3.15 | 3.15 | 3.15 | 3.15 | 3.15 |
| Soybean oil/cyclosporin A | 25.00 | 9.00 | 5.2 | 4.34 | 3.52 | 2.60 |
| Transparency | Transparent | Transparent | Transparent | Transparent | Transparent | Transparent |
| Stability/precipitation (days) | > 14 | > 14 | > 14 | > 14 | > 14 | Deposition on 8th day |
| Average particle diameter (nm) | 11.86 | 10.42 | 11.35 | 11.43 | 10.95 | 11.44 |
| Particle size distribution index PDI | 0.069 | 0.077 | 0.042 | 0.094 | 0.038 | 0.041 |
| Subpeak of average particle diameter of not less than 100 nm (%) | 0 | 0 | 0 | 0 | 0 | 0 |

**[Table 6]**

| | Example 24 | Example 25 | Example 26 |
|---|---|---|---|
| Water (% by weight) | 73.33 | 73.27 | 73.20 |
| Polysorbate 80 (% by weight) | 24.00 | 24.00 | 24.00 |
| Soybean oil (% by weight) | 2.00 | 2.00 | 2.00 |
| DDT (% by weight) | 0.67 | 0.73 | 0.80 |
| Polysorbate 80/soybean oil | 12.00 | 12.00 | 12.00 |
| Polysorbate 80/(soybean oil + DDT) | 8.99 | 8.79 | 8.57 |
| LogP of DDT | 6.50 | 6.50 | 6.50 |
| -0.38 × [LogP] + 5.169 | 2.70 | 2.70 | 2.70 |
| Soybean oil/DDT | 2.99 | 2.74 | 2.50 |
| Transparency | Transparent | Transparent | Transparent |
| Stability/precipitation (days) | >14 | > 14 | Deposition on 5th day |
| Average particle diameter (nm) | 11.24 | 10.82 | 11.19 |
| Particle size distribution index PDI | 0.053 | 0.031 | 0.037 |
| Subpeak of average particle diameter of not less than 100 nm (%) | 0 | 0 | 0 |

**[Table 7]**

| | Example 27 | Example 28 | Example 29 |
|---|---|---|---|
| Water (% by weight) | 72.25 | 72.15 | 72.19 |
| Polysorbate 80 (% by weight) | 25.00 | 25.00 | 25.00 |
| Soybean oil (% by weight) | 2.50 | 2.50 | 2.50 |
| Testosterone (% by weight) | 0.25 | 0.35 | 0.31 |
| Polysorbate 80/soybean oil | 10.00 | 10.00 | 10.00 |
| Polysorbate 80/(soybean oil + testosterone) | 9.09 | 8.77 | 8.90 |
| LogP of testosterone | 2.99 | 2.99 | 2.99 |
| -4.955 × [LogP] + 23.56 | 8.74 | 8.74 | 8.74 |
| Soybean oil/testosterone | 10.00 | 7.14 | 8.06 |
| Transparency | Transparent | Transparent | Transparent |
| Stability/precipitation (days) | >14 | Deposition on 2nd day | Deposition on 1st day |
| Average particle diameter (nm) | 12.44 | 11.63 | 10.62 |
| Particle size distribution index PDI | 0.118 | 0.042 | 0.057 |
| Subpeak of average particle diameter of not less than 100 nm (%) | 0 | 0 | 0 |

**[Table 8]**

| | Example 30 |
|---|---|
| Water (% by weight) | 72.18 |
| Polysorbate 80 (% by weight) | 25.00 |
| Soybean oil (% by weight) | 2.50 |
| Paclitaxel (% by weight) | 0.32 |
| Polysorbate 80/soybean oil | 10.00 |
| Polysorbate 80/(soybean oil + paclitaxel) | 8.87 |
| LogP of paclitaxel | 3.20 |
| -4.955 × [LogP] + 23.56 | 7.70 |
| Soybean oil/paclitaxel | 7.81 |

| Transparency | Transparent |
|---|---|
| Stability/precipitation (days) | > 14 |
| Average particle diameter (nm) | 11.12 |
| Particle size distribution index PDI | 0.047 |
| Subpeak of average particle diameter of not less than 100 nm (%) | 0 |

**[Table 9]**

| | Example 31 | Example 32 |
|---|---|---|
| Water (% by weight) | 73.20 | 73.15 |
| Polysorbate 80 (% by weight) | 24.00 | 24.00 |
| Soybean oil (% by weight) | 2.60 | 2.60 |
| Menthol (% by weight) | 0.20 | 0.25 |
| Polysorbate 80/soybean oil | 9.23 | 9.23 |
| Polysorbate 80/(soybean oil + menthol) | 8.57 | 8.42 |
| LogP of menthol | 2.68 | 2.68 |
| -4.955 × [LogP] + 23.56 | 10.28 | 10.28 |
| Soybean oil/menthol | 12.89 | 10.38 |

| Transparency | Transparent | Transparent |
|---|---|---|
| Stability/precipitation (days) | > 14 | > 14 |
| Average particle diameter (nm) | 11.20 | 11.81 |
| Particle size distribution index PDI | 0.048 | 0.056 |
| Subpeak of average particle diameter of not less than 100 nm (%) | 0 | 0 |

**[Table 10]**

| | Example 33 | Example 34 |
|---|---|---|
| Water (% by weight) | 72.88 | 72.78 |
| Polysorbate 80 (% by weight) | 24.00 | 23.94 |
| Soybean oil (% by weight) | 2.08 | 2.28 |
| Tocopherol (% by weight) | 1.04 | 1.00 |
| Polysorbate 80/soybean oil | 11.54 | 10.50 |
| Polysorbate 80/(soybean oil + tocopherol) | 7.69 | 7.30 |
| LogP of tocopherol | 8.84 | 8.84 |
| -0.38 × [LogP] + 5.169 | 1.81 | 1.81 |
| Soybean oil/tocopherol | 2.00 | 2.28 |

| Transparency | Transparent | Transparent |
|---|---|---|
| Stability/precipitation (days) | > 14 | > 14 |
| Average particle diameter (nm) | 10.85 | 11.15 |
| Particle size distribution index PDI | 0.035 | 0.104 |
| Subpeak of average particle diameter of not less than 100 nm (%) | 0 | 0 |

**[Table 11]**

| | Example 35 |
|---|---|
| Water (% by weight) | 73.91 |
| Polysorbate 80 (% by weight) | 23.94 |
| Soybean oil (% by weight) | 1.50 |
| Tocotrienol (% by weight) | 0.65 |
| Polysorbate 80/soybean oil | 15.96 |
| Polysorbate 80/(soybean oil + tocotrienol) | 11.15 |
| LogP of tocotrienol | 7.68 |
| -0.38 × [LogP] + 5.169 | 2.25 |
| Soybean oil/tocotrienol | 2.32 |

| Transparency | Transparent |
|---|---|
| Stability/precipitation (days) | > 14 |
| Average particle diameter (nm) | 9.96 |
| Particle size distribution index PDI | 0.016 |
| Subpeak of average particle diameter of not less than 100 nm (%) | 0 |

**[Table 12]**

| | Example 36 | Example 37 | Example 38 |
|---|---|---|---|
| Water (% by weight) | 74.22 | 74.08 | 73.88 |
| Polysorbate 80 (% by weight) | 24.00 | 24.00 | 24.00 |
| Soybean oil (% by weight) | 1.50 | 1.50 | 1.50 |
| Indomethacin (% by weight) | 0.38 | 0.41 | 0.62 |
| Polysorbate 80/soybean oil | 16.00 | 16.00 | 16.00 |
| Polysorbate 80/(soybean oil + indomethacin) | 12.77 | 12.57 | 11.32 |
| LogP of indomethacin | 4.25 | 4.25 | 4.25 |
| -0.38 × [LogP] + 5.169 | 3.55 | 3.55 | 3.55 |
| Soybean oil/indomethacin | 3.95 | 3.66 | 2.42 |
| Transparency | Transparent | Transparent | Transparent |
| Stability/precipitation (days) | > 14 | > 14 | Deposition on 1st day |
| Average particle diameter (nm) | 9.06 | 8.959 | 8.469 |
| Particle size distribution index PDI | 0.06 | 0.046 | 0.031 |
| Subpeak of average particle diameter of not less than 100 nm (%) | 0 | 0 | 0 |

As shown in Tables 1 to 12, it can be seen that in a case where the weight ratio of the polysorbate 80 to the oil is not less than 9.20 and not more than 240, and the weight ratio of the polysorbate 80 to the sum of the oil and the poorly water-soluble functional component is not less than 5.20 and not more than 200, it is possible to obtain a microemulsion containing a monodisperse poorly water-soluble drug such that the average particle diameter of the emulsion particles of the obtained microemulsion is not more than 18 nm, the PDI (particle size distribution index) is not more than 0.14, and the microemulsion is a monodisperse microemulsion which does not contain particles of not less than 100 nm (subpeaks).

Further, from Tables 2 and 3, it can be seen that the average particle diameter tends to increase as the weight ratio of the polysorbate 80 to the oil falls below 9.20 and approaches 4.

Further, as shown in Tables 1 to 12, it can be seen that in a case where: the weight ratio of the polysorbate 80 to the oil is not less than 9.20 and not more than 240; the weight ratio of the polysorbate 80 to the sum of the oil and the poorly water-soluble functional component is not less than 5.20 and not more than 200; when LogP of the poorly water-soluble functional component is not less than 4.2, a weight ratio of the oil to the poorly water-soluble functional component is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000, it is possible to obtain a stable microemulsion containing a monodisperse poorly water-soluble drug such that the average particle diameter of the emulsion particles of the obtained microemulsion is not more than 18 nm, the PDI (particle size distribution index) is not more than 0.14, and the microemulsion is a monodisperse microemulsion which does not contain particles of not less than 100 nm (subpeaks).

That is, it can be seen that in a case where: when LogP of the poorly water-soluble functional component is not less than 4.2, the weight ratio of the oil to the poorly water-soluble functional component is not less than [-0.38 × (the LogP of the poorly water-soluble functional component) + 5.169] and not more than 2000; and when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000, no drug deposition occurs, and more excellent stability is exhibited, in comparison with Examples 12, 26, and 38 in which when the LogP of the poorly water-soluble functional component is not less than 4.2, the weight ratio of the oil to the poorly water-soluble functional component is less than [-0.38 × (the LogP of the poorly water-soluble functional component) + 5.169] and Examples 23, 28, and 29 in which when the LogP of the poorly water-soluble functional component is less than 4.2, the weight ratio of the oil to the poorly water-soluble functional component is less than [-4.955 × (the LogP of the poorly water-soluble functional component) + 23.56].

### (Examples 39 and 40)

Microemulsions were each prepared in the same manner as in Example 1 except that a plurality of poorly water-soluble functional components shown in Table 13 were used as the poorly water-soluble functional component, oils shown in Table 13 were used as the oil, and the weight ratio of the poorly water-soluble functional component, the oil, the polysorbate 80, and the ion-exchanged water was changed to the weight ratios shown in Table 13. The obtained microemulsions were evaluated. The results are shown in Table 13. In Example 40, the following oil was used as the oil.

Squalane (available from Oryza Oil & Fat Chemical Co., Ltd.; ORYZA SQUALANE)

**[Table 13]**

| | | Example 39 | Example 40 |
|---|---|---|---|
| Water (% by weight) | | 71.70 | 71.70 |
| Polysorbate 80 (% by weight) | | 25.00 | 25.00 |
| Oil | Soybean oil (% by weight) | 2.50 | |
| | Squalane | | 2.50 |
| Functional component | CoQ10 | 0.50 | 0.50 |
| | Curcumin | 0.20 | 0.20 |
| | Tocopherol | 0.10 | 0.10 |
| Polysorbate 80/oil | | 10.00 | 10.00 |
| Polysorbate 80/(oil + functional component) | | 7.58 | 7.58 |
| LogP of CoQ10 | | 11 | 11 |
| -0.38 × [LogP] + 5.169 | | 0.99 | 0.99 |
| Oil (0.6)jCoQ10 (0.5) | | 1.20 | 1.20 |
| LogP of curcumin | | 3.62 | 3.62 |
| -4.955 × [LogP] + 23.56 | | 5.62 | 5.62 |
| Oil (1.6)/curcumin (0.2) | | 8.00 | 8.00 |
| LogP of tocopherol | | 8.84 | 8.84 |
| -0.38 × [LogP] + 5.169 | | 1.81 | 1.81 |
| Oil (0.3)/tocopherol (0.1) | | 3.00 | 3.00 |
| Transparency | | Transparent | Transparent |
| Stability/precipitation (days) | | > 14 | > 14 |
| Average particle diameter (nm) | | 13.04 | 13.02 |
| Particle size distribution index PDI | | 0.033 | 0.036 |
| Subpeak of average particle diameter of not less than 100 nm (%) | | 0 | 0 |

As shown in Table 13, in Examples 39 and 40, three types of poorly water-soluble functional components were used in combination. Since the amount of oil required varies depending on the type (LogP value) of poorly water-soluble functional component, the amount obtained by dividing the amount of oil used (2.50% by weight) for each of the poorly water-soluble functional components was used to calculate the weight ratio of the oil to the poorly water-soluble functional components. As shown in Table 13, even when a plurality of types of poorly water-soluble functional components are used in combination, it can be seen that in a case where: the weight ratio of the polysorbate 80 to the oil is not less than 9.20 and not more than 240; the weight ratio of the polysorbate 80 to the sum of the oil and the poorly water-soluble functional component is not less than 5.20 and not more than 200; when LogP of the poorly water-soluble functional component is not less than 4.2, a weight ratio of the oil to each of the poorly water-soluble functional components is not less than [-0.38 × (the LogP of the poorly water-soluble functional component) + 5.169] and not more than 2000; and when the LogP of the poorly water-soluble functional component is less than 4.2, the weight ratio of the oil to each of the poorly water-soluble functional components is not less than [-4.955 × (the LogP of the poorly water-soluble functional component) + 23.56] and not more than 2000, it is possible to obtain a stable microemulsion containing a monodisperse poorly water-soluble drug such that the average particle diameter of the emulsion particles of the obtained microemulsion is not more than 18 nm, the PDI (particle size distribution index) is not more than 0.14, and the microemulsion is a monodisperse microemulsion which does not contain particles of not less than 100 nm (subpeaks).

### (Examples 41 to 46)

Microemulsions were prepared in the same manner as in Example 1 except that curcumin was used as the poorly water-soluble functional component, a soybean oil or medium chain fatty acid triglyceride was used as the oil, a polyoxyethylene castor oil was used alone or in combination with polysorbate 80, as a surfactant component, and the weight ratio of the poorly water-soluble functional component, the oil, the surfactant component, and the ion-exchanged water was changed to the weight ratios shown in Table 14. The obtained microemulsions were evaluated. The results are shown in Table 14. In Example 41, the polyoxyethylene castor oil was used alone as the surfactant component. In Examples 42 to 46, the polyoxyethylene castor oil and the polysorbate 80 were used in combination as the surfactant component. Further, in Examples 45 and 46, the medium chain fatty acid triglyceride was used as the oil. The following oils were used as the oil, and the following surfactants were used as the surfactant.
Medium chain fatty acid triglyceride (available from Kao Corporation; trade name: CO-CONARD RK)
Polyoxyethylene castor oil (available from BASF; Kolliphor (registered trademark) EL)

**[Table 14]**

| | | Example 41 | Example 42 | Example 43 | Example 44 | Example 45 | Example 46 |
|---|---|---|---|---|---|---|---|
| Water (% by weight) | | 73.2 | 73.2 | 73.2 | 67.5 | 73.2 | 67.5 |
| Surfactant component | Polyoxyethylene castor oil (% by weight) | 24.0 | 19.0 | 5.0 | 24.0 | 19.0 | 24.0 |
| | Polysorbate 80 (% by weight) | | 5.0 | 19.0 | 5.0 | 5.0 | 5.0 |
| Oil | Soybean oil (% by weight) | 2.4 | 2.4 | 2.4 | 3.0 | | |
| | Medium chain fatty acid triglyceride (% by weight) | | | | | 2.4 | 3 |
| Curcumin (% by weight) | | 0.4 | 0.4 | 0.4 | 0.5 | 0.4 | 0.5 |
| Surfactant component/oil | | 10.0 | 10.0 | 10.0 | 9.67 | 10.0 | 9.67 |
| Polysorbate 80/(oil + curcumin) | | 8.57 | 8.57 | 8.57 | 8.29 | 8.57 | 8.29 |
| LogP of curcumin | | 3.62 | 3.62 | 3.62 | 3.62 | 3.62 | 3.62 |
| -4.955 × [LogP] + 23.56 | | 5.62 | 5.62 | 5.62 | 5.62 | 5.62 | 5.62 |
| Oil/curcumin | | 6.00 | 6.00 | 6.00 | 6.00 | 6.00 | 6.00 |
| Transparency | | Transparent | Transparent | Transparent | Transparent | Transparent | Transparent |
| Stability/precipitation (days) | | > 14 | > 14 | > 14 | > 14 | > 14 | > 14 |
| Average particle diameter (nm) | | 15.46 | 14.41 | 11.52 | 14.42 | 12.34 | 13.09 |
| Particle size distribution index PDI | | 0.036 | 0.033 | 0.053 | 0.046 | 0.034 | 0.034 |
| Subpeak of average particle diameter of not less than 100 nm (%) | | 0 | 0 | 0 | 0 | 0 | 0 |

As shown in Table 14, it can be seen that in a case where: the weight ratio of the polyoxyethylene castor oil to the oil or the weight ratio of the surfactant component which is composed of both the polysorbate 80 and the polyoxyethylene castor oil to the oil is not less than 9.20 and not more than 240; the weight ratio of the polyoxyethylene castor oil to the sum of the oil and the poorly water-soluble functional component or the weight ratio of the surfactant component which is composed of both the polysorbate 80 and the polyoxyethylene castor oil to the sum of the oil and the poorly water-soluble functional component is not less than 5.20 and not more than 200; the weight ratio of the oil to the poorly water-soluble functional component is not less than [-4.955 × (the LogP of the poorly water-soluble functional component) + 23.56] and not more than 2000, it is possible to obtain a stable microemulsion containing a monodisperse poorly water-soluble drug such that the average particle diameter of the emulsion particles of the obtained microemulsion is not more than 18 nm, the PDI (particle size distribution index) is not more than 0.14, and the microemulsion is a monodisperse microemulsion which does not contain particles of not less than 100 nm (subpeaks).

### Industrial Applicability

A composition for preparing a microemulsion and a microemulsion in accordance with an embodiment of the present invention each have the above-described arrangements, so that it is possible to provide a microemulsion having emulsion particles of a small particle diameter, having a narrow particle diameter distribution, having improved uniformity in particle diameter of the emulsion particles, and having excellent stability. Therefore, the composition for preparing a microemulsion and the microemulsion in accordance with an embodiment of the present invention are applicable in the fields of using the microemulsion, including functional foods, such as pharmaceutical products and supplements, cosmetic products, coating agents, and the like.

## Claims

1. A microemulsion comprising:
- a composition, the composition comprising:
- a surfactant component which contains at least one selected from polysorbate 80 and a polyoxyethylene castor oil;
- an oil; and
- a poorly water-soluble functional component,
wherein
- the surfactant component contains at least one selected from the polysorbate 80 and the polyoxyethylene castor oil in an amount of not less than 80% by weight relative to a total weight of the surfactant component,
- the microemulsion is an optically transparent or semi-transparent dispersion system of emulsion particles having a particle diameter of several nanometers to several tens of nanometers, wherein the particle diameter is measured as described in section Evaluation method of the description,
- the oil is an oil having a freezing point of not higher than 25°C,
- the poorly water-soluble functional component has solubility in water at 20°C of not more than 10 mg/ml,
- a weight ratio of the surfactant component to the oil is not less than 9.20 and not more than 240,
- a weight ratio of the surfactant component to a sum of the oil and the poorly water-soluble functional component is not less than 5.20 and not more than 200,
- when LogP of the poorly water-soluble functional component is not less than 4.2, a weight ratio of the oil to the poorly water-soluble functional component is not less than [-0.38 × (the LogP of the poorly water-soluble functional component) + 5.169] and not more than 2000, and
- when the LogP of the poorly water-soluble functional component is less than 4.2, the weight ratio of the oil to the poorly water-soluble functional component is not less than [-4.955 × (the LogP of the poorly water-soluble functional component) + 23.56] and not more than 2000; and
- an aqueous medium,
wherein:
- the amount of the aqueous medium contained in the microemulsion is not less than 50% by weight.

2. A pharmaceutical product, a functional food, a cosmetic product, a coating agent, or a pesticide, each comprising a microemulsion according to claim 1.

3. A method for producing the microemulsion of claim 1, the method comprising:
- a mixing step of mixing a surfactant component which contains at least one selected from polysorbate 80 and a polyoxyethylene castor oil, an oil, and a poorly water-soluble functional component,
wherein:
- the surfactant component contains at least one selected from the polysorbate 80 and the polyoxyethylene castor oil in an amount of not less than 80% by weight relative to a total weight of the surfactant component,
- a weight ratio of the surfactant component to the oil is not less than 9.20 and not more than 240, and
- a weight ratio of the surfactant component to a sum of the oil and the poorly water-soluble functional component is not less than 5.20 and not more than 200; and
- a step of mixing a composition obtained in the mixing step and an aqueous medium, wherein
- the amount of the aqueous medium contained in the microemulsion is not less than 50% by weight.

4. The microemulsion according to claim 1, the microemulsion having emulsion particles having an average particle diameter of not more than 18 nm and having a particle size distribution index of not more than 0.14,
- each of the average particle diameter and the particle size distribution index of the emulsion particles being a value that is measured with use of Zetasizer Nano ZS, available from Malvern Institutes, in a measurement mode *"size-small-vol-cell*×*1.SOP",* and
- the microemulsion being a monodisperse microemulsion which does not contain particles of not less than 100 nm.

## Patentansprüche

1. Mikroemulsion, umfassend:
- eine Zusammensetzung, wobei die Zusammensetzung umfasst:
- eine Tensidkomponente, die mindestens eines ausgewählt aus Polysorbat 80 und einem Polyoxyethylen-Rizinusöl enthält;
- ein Öl; und
- eine schwer wasserlösliche funktionelle Komponente,
wobei
- die Tensidkomponente mindestens eines ausgewählt aus dem Polysorbat 80 und dem Polyoxyethylen-Rizinusöl in einer Menge von nicht weniger als 80 Gew.-%, bezogen auf ein Gesamtgewicht der Tensidkomponente, enthält,
- die Mikroemulsion ein optisch transparentes oder halbtransparentes Dispersionssystem von Emulsionsteilchen mit einem Teilchendurchmesser von mehreren Nanometern bis mehreren zehn Nanometern ist, wobei der Teilchendurchmesser wie in Abschnitt Evaluierungsverfahren der Beschreibung beschrieben gemessen wird,
- das Öl ein Öl mit einem Gefrierpunkt von nicht höher als 25 °C ist,
- die schwer wasserlösliche funktionelle Komponente eine Löslichkeit in Wasser bei 20 °C von nicht mehr als 10 mg/ml aufweist,
- ein Gewichtsverhältnis der Tensidkomponente zu dem Öl nicht weniger als 9,20 und nicht mehr als 240 beträgt,
- ein Gewichtsverhältnis der Tensidkomponente zu einer Summe des Öls und der schwer wasserlöslichen funktionellen Komponente nicht weniger als 5,20 und nicht mehr als 200 beträgt,
- wenn LogP der schwer wasserlöslichen funktionellen Komponente nicht weniger als 4,2 beträgt, ein Gewichtsverhältnis des Öls zu der schwer wasserlöslichen funktionellen Komponente nicht weniger als [-0,38 × (der LogP der schwer wasserlöslichen funktionellen Komponente) + 5,169] und nicht mehr als 2000 beträgt, und
- wenn der LogP der schwer wasserlöslichen funktionellen Komponente weniger als 4,2 beträgt, das Gewichtsverhältnis des Öls zu der schwer wasserlöslichen funktionellen Komponente nicht weniger als [-4,955 × (der LogP der schwer wasserlöslichen funktionellen Komponente) + 23,56] und nicht mehr als 2000 beträgt; und
- ein wässriges Medium,
wobei:
- die Menge des in der Mikroemulsion enthaltenen wässrigen Mediums nicht weniger als 50 Gew.-% beträgt.

2. Pharmazeutisches Produkt, funktionelles Lebensmittel, kosmetisches Produkt, Beschichtungsmittel oder Pestizid, jeweils umfassend eine Mikroemulsion nach Anspruch 1.

3. Verfahren zur Herstellung der Mikroemulsion nach Anspruch 1, wobei das Verfahren umfasst:
- einen Mischschritt des Mischens einer Tensidkomponente, die mindestens eines ausgewählt aus Polysorbat 80 und einem Polyoxyethylen-Rizinusöl enthält, eines Öls und einer schwer wasserlöslichen funktionellen Komponente,
wobei:
- die Tensidkomponente mindestens eines ausgewählt aus dem Polysorbat 80 und dem Polyoxyethylen-Rizinusöl in einer Menge von nicht weniger als 80 Gew.-%, bezogen auf ein Gesamtgewicht der Tensidkomponente, enthält,
- ein Gewichtsverhältnis der Tensidkomponente zu dem Öl nicht weniger als 9,20 und nicht mehr als 240 beträgt, und
- ein Gewichtsverhältnis der Tensidkomponente zu einer Summe des Öls und der schwer wasserlöslichen funktionellen Komponente nicht weniger als 5,20 und nicht mehr als 200 beträgt; und
- einen Schritt des Mischens einer Zusammensetzung, erhalten in dem Mischschritt, und ein wässriges Medium,
wobei
- die Menge des in der Mikroemulsion enthaltenen wässrigen Mediums nicht weniger als 50 Gew.-% beträgt.

4. Mikroemulsion nach Anspruch 1, wobei die Mikroemulsion Emulsionsteilchen mit einem mittleren Teilchendurchmesser von nicht mehr als 18 nm und mit einem Teilchengrößenverteilungsindex von nicht mehr als 0,14 aufweist,
- wobei der mittlere Teilchendurchmesser und der Teilchengrößenverteilungsindex der Emulsionsteilchen jeweils ein Wert sind, der unter Verwendung von Zetasizer Nano ZS, erhältlich von Malvern Institutes, in einem Messmodus "*size-small-vol-cell x 1.SOP"* gemessen wird, und
- wobei die Mikroemulsion eine monodisperse Mikroemulsion ist, die nicht Teilchen von nicht weniger als 100 nm enthält.

## Revendications

1. Microémulsion comprenant :
- une composition, la composition comprenant :
- un composant tensioactif qui contient au moins un élément choisi parmi le polysorbate 80 et une huile de ricin polyoxyéthylénée ;
- une huile ; et
- un composant fonctionnel peu soluble dans l'eau,
dans laquelle
- le composant tensioactif contient au moins un élément choisi parmi le polysorbate 80 et l'huile de ricin polyoxyéthylénée en une quantité d'au moins 80 % en poids par rapport à un poids total du composant tensioactif,
- la microémulsion est un système de dispersion optiquement transparent ou semi-transparent de particules d'émulsion ayant un diamètre de particule compris entre plusieurs nanomètres et plusieurs dizaines de nanomètres, dans laquelle le diamètre des particules est mesuré comme décrit dans la section « Méthode d'évaluation » de la description,
- l'huile est une huile ayant un point de congélation ne dépassant pas 25 °C,
- le composant fonctionnel peu soluble dans l'eau présente une solubilité dans l'eau à 20 °C ne dépassant pas 10 mg/mL,
- un rapport pondéral entre le composant tensioactif et l'huile n'est pas inférieur à 9,20 et n'est pas supérieur à 240,
- un rapport pondéral entre le composant tensioactif et la somme de l'huile et du composant fonctionnel peu soluble dans l'eau n'est pas inférieur à 5,20 et n'est pas supérieur à 200,
- lorsque le LogP du composant fonctionnel peu soluble dans l'eau n'est pas inférieur à 4,2, un rapport pondéral entre l'huile et le composant fonctionnel peu soluble dans l'eau n'est pas inférieur à [-0,38 × (le LogP du composant fonctionnel peu soluble dans l'eau) + 5,169] et n'est pas supérieur à 2 000, et
- lorsque le LogP du composant fonctionnel peu soluble dans l'eau est inférieur à 4,2, le rapport pondéral entre l'huile et le composant fonctionnel peu soluble dans l'eau est compris entre [-4,955 × (le LogP du composant fonctionnel peu soluble dans l'eau) + 23,56] et 2 000 ; et
- un milieu aqueux,
dans laquelle :
- la quantité de milieu aqueux contenue dans la microémulsion n'est pas inférieure à 50 % en poids.

2. Produit pharmaceutique, aliment fonctionnel, produit cosmétique, agent d'enrobage ou pesticide, chacun comprenant une microémulsion selon la revendication 1.

3. Procédé de fabrication de la microémulsion selon la revendication 1, le procédé comprenant :
- une étape de mélange consistant à mélanger un composant tensioactif qui contient au moins un élément choisi parmi le polysorbate 80 et une huile de ricin polyoxyéthylénée, une huile et un composant fonctionnel peu soluble dans l'eau,
dans laquelle :
- le composant tensioactif contient au moins un élément choisi parmi le polysorbate 80 et l'huile de ricin polyoxyéthylénée en une quantité non inférieure à 80 % en poids par rapport au poids total du composant tensioactif,
- un rapport pondéral entre le composant tensioactif et l'huile n'est pas inférieur à 9,20 et n'est pas supérieur à 240, et
- un rapport pondéral entre le composant tensioactif et la somme de l'huile et du composant fonctionnel peu soluble dans l'eau n'est pas inférieur à 5,20 et n'est pas supérieur à 200 ; et
- une étape consistant à mélanger une composition obtenue lors de l'étape de mélange et un milieu aqueux,
dans lequel
- la quantité de milieu aqueux contenue dans la microémulsion n'est pas inférieure à 50 % en poids.

4. Microémulsion selon la revendication 1, la microémulsion comportant des particules d'émulsion ayant un diamètre moyen de particule ne dépassant pas 18 nm et présentant un indice de distribution granulométrique ne dépassant pas 0,14,
- le diamètre moyen de particule et l'indice de distribution granulométrique des particules d'émulsion étant chacun une valeur mesurée à l'aide du Zetasizer Nano ZS, disponible auprès de Malvern Institutes, en mode de mesure « *size-small-vol-cell x 1. SOP* », et
- la microémulsion étant une microémulsion monodispersée qui ne contient pas de particules ayant un diamètre d'au moins 100 nm.
